# EUROPEAN PATENT APPLICATION

(11) **EP 1 700 586 A2**
(43) Date of publication of application: **13.09.2006**
(21) Application number: 06003667.0
(22) Date of filing: 23.02.2006
(51) Int. Cl.: A61F 13/15

(54) **Method of making absorbent structures with encapsulated superabsorbent material**

(30) Priority: 11.03.2005 US 906908
(71) Applicant: NORDSON CORPORATION, Westlake, Ohio 44145-1119 (US)
(72) Inventor: Bentley, Rachelle, Cumming,Georgia 30040 (US); Crane, Patrick L., Dawsonville,Georgia 30534 (US); Bernal, Stephen D., Cincinnati 45240, Ohio (US); Davis, James H., Amelia 45102, Ohio (US); Malakouti, Nezam, Loveland 45140, Ohio (US)
(74) Representative: Eisenführ, Speiser & Partner

(57) **Abstract**

A method of making an absorbent core structure includes meltspinning at least a first layer of fibrous material having a plurality of first portions and a plurality of second portions. A superabsorbent material is deposited between the respective first and second portions of the first layer. The first portions of the first layer are moved with respect to the second portions of the first layer so as to at least substantially encapsulate the deposited superabsorbent material between the respective first and second portions.

## Description

### FIELD OF THE INVENTION

The present invention relates to absorbent core structures for disposable absorbent articles. More specifically, the present invention relates to absorbent core structures constructed of fibrous materials.

### BACKGROUND OF THE INVENTION

Disposable absorbent articles having absorbent core structures are well known in the art. Furthermore, it is well known that such absorbent core structures have at least three functional regions, namely, an acquisition region, a distribution region, and a storage region. While such regions are known, the design of absorbent core structures having said regions is limited by current methods of manufacture and current material selections.

One such conventional absorbent core structure includes the use of cellulosic materials. While the use of cellulosic materials provides satisfactory acquisition and distribution, often cellulosic core structures suffer from having poor wet integrity (i.e., has poor structural integrity when wet). In an effort to improve the wet integrity of such cellulosic core structures, the incorporation of expensive binders is often used. Another known problem when using cellulosic materials is the presence of knots and fines which are unsatisfactorily shaped fibers that negatively impact the core properties (e.g., efficacy, cost).

Another such conventional absorbent core structure includes the use of synthetic meltblown fibers. While the use of synthetic meltblown fibers may provide satisfactory wet integrity with the use of binders, the resulting core structure is often limited in design. For example, synthetic meltblown fibers are generally small in diameter (e.g., 2 - 9 microns); thus, the resulting core structure would generally have poor acquisition properties. Further, these smaller fibers tend to be weak thus not permitting the creation of post-hydrated void areas.

It is also well known that conventional absorbent core structures for use in disposable absorbent articles may be made of discrete, multiple layers of materials. Further, it is well known that said layers may consist of different types of materials. For example, a conventional absorbent article may be made of: (a) a top layer which serves as an acquisition region for more immediate absorption of exudate from the wearer, (b) an intermediate layer which serves as a distribution region for the intended transportation of exudate within the absorbent core structure (e.g., move exudate longitudinally or laterally for greater utilization of diaper) and (c) a bottom layer which serves as a storage region for more long-term storage of exudate. However, the conventional use of discrete, multiple layers often results in poor fluid communication between said layers.

What is needed is an absorbent core structure made of fibrous material in which properties of the acquisition region, distribution region, and storage region can be easily varied in the vertical and/or horizontal direction.

### SUMMARY OF THE INVENTION

An absorbent core structure having at least one acquisition region, at least one distribution region, and at least one storage region. The acquisition region being constructed from a fibrous material. The acquisition region having a relatively low density from about 0.018 g/cc to about 0.20 g/cc. The at least one distribution region being constructed from the fibrous material. The distribution region being consolidated to have a relatively medium density from about 0.024 g/cc to about 0.45 g/cc. The distribution region being in fluid communication with the acquisition region. The at least one storage region being constructed from the fibrous material. The storage region being consolidated to have a relatively high density from about 0.030 g/cc to about 0.50 g/cc. The storage region being in fluid communication with the distribution region. A portion of the fibrous material being formed into at least one peak and at least one valley and then subsequently closed on itself in order to cause the peaks to close to form insitubes.

The fibrous material may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as a superabsorbent polymer (SAP) and/or other material having superabsorbent properties. The SAP may be deposited onto the at least one of the valley. The SAP may be deposited onto the at least one of the peak. The SAP may be deposited onto the at least one of the valley and onto the at least one of the peak. The SAP may be deposited onto alternating valleys. The SAP may be deposited onto alternating peaks. The absorbent core structure may also include a retractable material which is applied to the peaks. The retractable material retracts upon the introduction of a stimulus which causes the peaks to also retract in order to close the valleys to form the insitubes. The retractable material may be a polyester. The retractable material may be an elastic strand. The retractable material may be applied to the peaks by use of an adhesive. The adhesive may be applied continuously onto the fibrous material. The adhesive may be applied discontinuously onto the fibrous material.

An absorbent core structure having at least one acquisition region, at least one distribution region, at least one storage region and a SAP. The acquisition region being constructed from a fibrous material. The acquisition region having a relatively low density from about 0.018 g/cc to about 0.20 g/cc. The distribution region being constructed from the fibrous material. The distribution region being consolidated to have a relatively medium density from about 0.024 g/cc to about 0.45 g/cc. The distribution region being in fluid communication with said acquisition region. The at least one storage region being constructed from the fibrous material. The storage region being consolidated to have a relatively high density from about 0.030 g/cc to about 0.50 g/cc. The storage region being in fluid communication with the distribution region.

A portion of the fibrous material may be formed into at least one insitube. The insitube may be formed by the folding-over of a plurality of filaments of the fibrous material in such a way as to encapsulate the SAP. The fibrous material may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof.

The invention further contemplates various methods of making an absorbent core structure, such as for use in a disposable hygienic product. In one illustrative embodiment, the method of making an absorbent core structure from a layer of fibrous material comprises meltspinning at least a first layer of fibrous material having a plurality of first portions and a plurality of second portions. The meltspinning process may, for example, involve meltblowing and/or spunbonding processes that deposit fibers on a moving collector such as a conveying element formed from wire. A superabsorbent material, such as those formed from various polymers and/or other materials, is deposited between the respective first and second portions of the first layer. The first portions of the first layer are moved, in various possible manners, with respect to the second portions of the first layer so as to at least substantially encapsulate the deposited superabsorbent material between the respective first and second portions.

In one embodiment, the first and second portions are respectively formed as peaks and valleys and the method further comprises depositing the superabsorbent material in at least the valleys. The superabsorbent material may alternatively be generally uniformly deposited across the peaks and valleys. Moving the first portions of the first layer with respect to the second portions of the first layer may be accomplished in various manners. For example, the first layer of fibrous material may be connected to a contractable element and the contractable element may be caused to contract. The contractable element may, for example, further comprise a stretched elastic strand or a second fibrous layer that shrinks upon application of a stimulus, such as heat, etc. Moving the first portions of the first layer with respect to the second portions of the first layer so as to at least substantially encapsulate the deposited superabsorbent material may also involve forming the first and second portions generally into tubular structures with at least a portion of the superabsorbent material received within at least some of the tubular structures.

The superabsorbent material may be deposited onto the first layer of fibrous material with the first layer of fibrous material in various conditions, such as an undulating or peak/valley configuration, or a generally flat condition. In addition, the superabsorbent material may be deposited in generally continuous layer form, or as discrete, spaced apart amounts of material. In other aspects of the invention, one or more additional layers of fibrous material may be secured together, or portions of layers may be folded over other portions.

The invention further contemplates various apparatus for manufacturing an absorbent core structure. In one illustrative embodiment, an apparatus comprises a web configuration device operative to receive a layer of fibrous material and form a plurality of peaks and valleys in the layer of fibrous material. An applicator device is positioned downstream of the web configuration device and is operative to deposit a superabsorbent material into at least the valleys in the layer of fibrous material. An encapsulation device is positioned downstream of the applicator device and operates to close the peaks against one another to thereby at least substantially encapsulate the superabsorbent material within the valleys.

The web configuration device may further comprise first and second rotary members engageable with opposite sides of the layer of fibrous material. The encapsulation device may further comprise third and fourth rotary members engageable with opposite sides of the layer of fibrous material. In accordance with this aspect, the third and fourth rotary members may be controlled to operate at a lower speed than the first and second rotary members so as to cause the peaks to close against one another.

Various additional features, advantages and objectives of the invention will become more readily apparent to those of ordinary skill in the art upon review of the following detailed description of the preferred embodiments taken in conjunction with the accompanying drawings.

### BRIEF DESCRIPTION OF THE DRAWINGS

FIG. 1 provides a block diagram of an exemplary manufacturing process in accordance with the present invention;

FIG. 2a provides a schematic of an exemplary manufacturing process using a roll in accordance with the present invention;

FIG. 2b provides a schematic of an exemplary manufacturing process using a belt in accordance with the present invention;

FIG. 3 shows an exemplary resulting product at position 100b within the manufacturing process of FIG. 2a and FIG. 2b;

FIG. 4a shows an exemplary resulting product at position 100c within the manufacturing process of FIG. 2a and FIG. 2b;

FIG. 4b shows an exemplary large-diameter fibrous material with SAP being deposited substantially along the entire surface of said large-diameter fibrous material;

FIG. 4c shows the product of FIG. 4b being substantially closed such that SAP is located inside the closed valleys and on top of the peaks;

FIG. 5a shows another exemplary resulting product at position 100c within the manufacturing process of FIG. 2a and FIG. 2b, wherein product includes an elastic member affixed to said large-diameter fibrous material;

FIG. 5b shows product of FIG. 5a where the peaks will substantially close to form tubes;

FIG. 5c alternate product wherein discrete applications of adhesive are applied to elastic member;

FIG. 5d shows product of FIG. 5c where the peaks will substantially close to form tubes;

FIG. 6a shows an exemplary large-diameter fibrous material in a substantially planar pre-condition having discrete depositions of SAP;

FIG. 6b shows product of FIG. 6a where peaks and valleys are formed;

FIG. 7a shows an exemplary large-diameter fibrous material in a substantially planar pre-condition having a substantially continuous deposition of SAP;

FIG. 7b shows product of FIG. 7a where peaks and valleys are formed;

FIG. 8a shows an exemplary large-diameter fibrous material having peaks and valleys with SAP deposited within said valleys;

FIG. 8b shows product of FIG. 8a where peaks and valleys are formed;

FIG. 9a shows an exemplary large-diameter fibrous material having peaks and valleys with SAP deposited within said valleys;

FIG. 9b shows product of FIG. 9a where peaks and valleys are formed;

FIG. 10a shows an exemplary large-diameter fibrous material which originally has a substantially planar shape;

FIG. 10b shows the product of FIG. 10a being formed to have a substantially closed tube around SAP;

FIG. 10c shows the product of FIG. 10b having a plurality of substantially closed tubes around SAP;

FIG. 10d shows the product of FIG. 10c being further consolidated;

FIG. 11 a shows a two-dimensional schematic view of an absorbent core having acquisition regions, distribution regions and storage regions being selectively placed throughout the core design;

FIG. 11b shows a three-dimensional schematic of FIG. 11a with fluid moving therein;

FIG. 11c shows a three-dimensional schematic of FIG. 11b with fluid moving further therein; and

FIG. 12 shows a three-dimensional schematic view of another absorbent core having acquisition regions, distribution regions and storage regions vary in their three-dimensional placement.

### DETAILED DESCRIPTION OF THE EMBODIMENTS

Various definitions of terms used herein are provided as follows:

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like. Said absorbent article may have an absorbent core having a garment surface and a body surface; a liquid permeable topsheet positioned adjacent said body surface of said absorbent core; and a liquid impermeable backsheet positioned adjacent said garment surface of said absorbent core.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

The term "diaper" herein refers to an absorbent article generally worn by infants and incontinent persons about the lower torso.

The term "pant", as used herein, refers to disposable garments having a waist opening and leg openings designed for infant or adult wearers.
A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The term "machine direction (MD)" or "longitudinal" herein refers to a direction running parallel to the maximum linear dimension of the article and/or fastening material and includes directions within ±45° of the longitudinal direction.

The term "cross direction (CD)", "lateral" or "transverse" herein refers to a direction which is orthogonal to the longitudinal direction.

The term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein the term "spunbond fibers" refers to small diameter fibers of substantially molecularly oriented polymeric material. Spunbond fibers are generally formed by extruding molten thermoplastic material as filaments from a plurality of fine, usually circular capillaries of a spinneret with the diameter of the extruded filaments then being rapidly reduced by an attenuation process. Spunbond fibers are generally not tacky when they are deposited onto a collecting surface and are generally continuous.

As used herein the term "spunbond material" refers to material made from spunbond fibers.

As used herein the term "meltblown fibers" means fibers of polymeric material which are generally formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter. Thereafter, the meltblown fibers can be carried by the high velocity gas stream and are deposited on a collecting surface to form a web of randomly dispersed meltblown fibers. Meltblown fibers may be continuous or discontinuous, are generally smaller than 10 microns in average diameter, and are generally tacky when deposited onto a collecting surface.

As used herein the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" includes all possible spatial configurations of the molecule. These configurations include, but are not limited to isotactic, syndiotactic and random symmetries.

As used herein, "ultrasonic bonding" means a process performed, for example, by passing the fabric between a sonic horn and anvil roll.

As used herein the term "acquisition layer" or "acquisition region" means a fibrous material having a relatively low density from about 0.018 g/cc to about 0.20 g/cc and a relatively high caliper from about 0.626 mm to about 5 mm.

As used herein the term "distribution layer" or "distribution region" means a fibrous material having a relatively medium density from about 0.024 g/cc to about 0.45 g/cc and a relatively medium caliper from about 0.34 mm to about 0.625 mm.

As used herein the terms "storage layer" or "storage region" mean any region that contains SAP. Further, said terms mean a fibrous material having a relatively high density from about 0.030 g/cc to about 0.50 g/cc and a relatively low caliper 0.33 mm to about 0.15 mm.

As used herein the term "small diameter" describes any fiber with a diameter of less than or equal to 10 microns.

As used herein the term "large diameter" describes any fiber with a diameter of greater than 10 microns.

As used herein the term "insitube" describes a corrugated or similar structure that may be sued to at least substantially encapsulate a material therein.

As used herein the term "superabsorbent" refers to a material that can absorb at least about 10 times its weight in fluid.

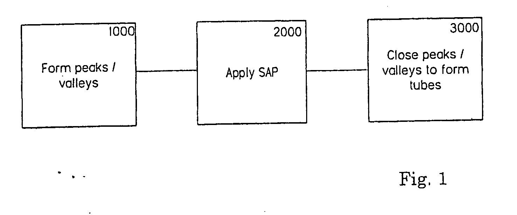

FIG. 1 provides a block diagram of an exemplary manufacturing process in accordance with the present invention. In a first step 1000, peaks and valleys are formed within a large-diameter fibrous material (e.g., spunbound material). In a second step 2000, super absorbent polymer (hereinafter SAP) is deposited in the valleys and/or peaks. In a third step 3000, the peaks are brought together to substantially close the valleys in order to form substantially closed regions (e.g., tubes).

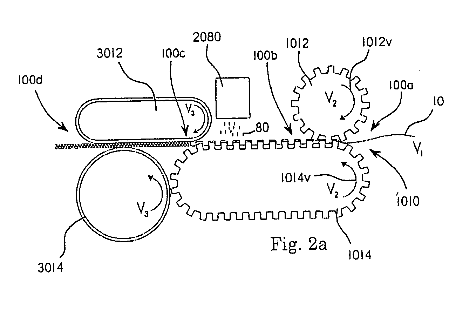

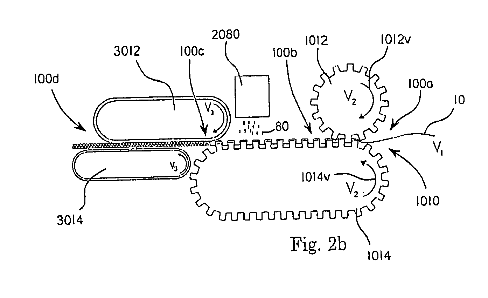

FIG. 2a provides a schematic of an exemplary manufacturing process in accordance with the present invention. Near a first position 100a, large-diameter fibrous material 10 is fed at a first velocity V₁ into a rotary nip 1010. The rotary nip 1010 may be comprised of a first rotary device 1012 and a second rotary device 1014 which rotate in opposing directions as indicated by arrows 1012v and 1014v, respectively. The rotary nip 1010 has a velocity V₂ which is less than or equal to velocity V₁. Near a second position 100b, large-diameter fibrous material 10 is formed within the nip such that peaks and valleys are created. Next, SAP applicator 2080 deposits SAP 80 into the recently formed peaks and/or valleys. Upon exiting the second rotary device 1014, the recently formed large-diameter fibrous material having SAP within its peaks and/or valleys is fed across a third rotary device 3012 near a third position 100c. The third rotary device 3012 has a velocity V₃ which is less than velocity V₂ such that the large-diameter fibrous material 10 begins to close on itself, thus causing the peaks to close to form an insitube. A fourth rotary device 3014 may be used in conjunction with the third rotary device to provide physical support of large-diameter fibrous material 10, metering capabilities of large-diameter fibrous material 10 and/or heat for bonding unconsolidated fibers. The fourth rotary device may be a roll (as shown in FIG. 2a), belt (as shown in FIG. 2b) or other suitable devices.

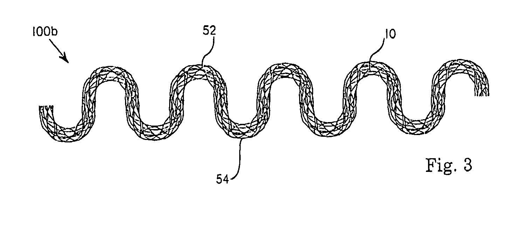

FIG. 3 shows an exemplary resulting product at position 100b within the manufacturing process of FIG. 2a and FIG. 2b. More specifically, FIG. 3 shows an exemplary large-diameter fibrous material 10 with peaks 52 and valleys 54 being formed therein. An exemplary basis weight for the large-diameter fibrous material may range from about 5 gsm to about 1000 gsm. An exemplary height of the peaks may range from about 1 mm to about 25 mm, and more preferably from about 3m to about 12 mm. The fibers of large-diameter fibrous material 10 may be made of a variety of suitable materials including, but not limited to, polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber having a large diameter, and combinations thereof. The large-diameter fibers of the present invention may have a diameter from about 10 micron to about 600 microns, unlike conventional meltblown fibers which typically have a diameter from about 2 to about 9 microns.

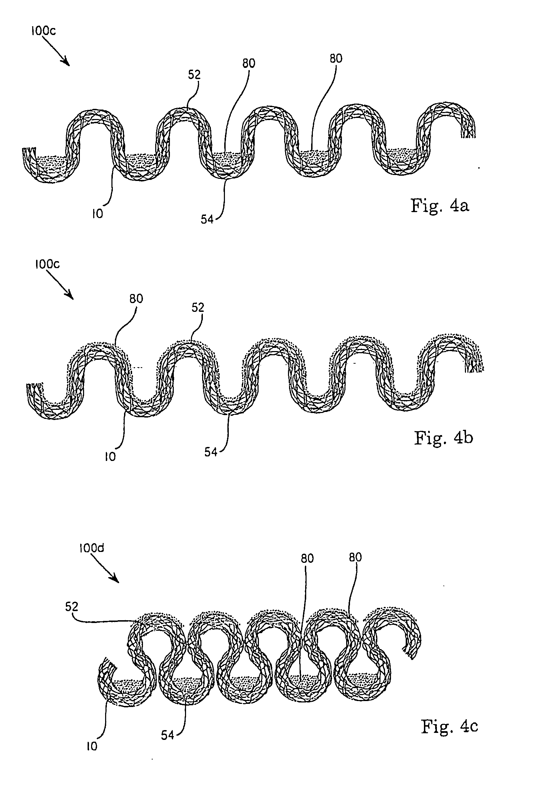

FIG. 4a shows an exemplary resulting product at position 100c within the manufacturing process of FIG. 2a and FIG. 2b. More specifically, FIG. 4a shows an exemplary large-diameter fibrous material 10 with SAP 80 being deposited within the valleys 54. Alternatively, FIG. 4b shows an exemplary large-diameter fibrous material 10 with SAP 80 being deposited substantially along the entire surface of the large-diameter fibrous material. FIG. 4c shows the product of FIG. 4b being substantially closed (i.e., as viewed at location 100d) such that SAP 80 is located inside the closed valleys 54 (i.e., tubes) and on top of the peaks 52. It may be desirable to surface treat (e.g., steam treatment, glue application, glycerine application, electrostatic treatment, microwave heating of fibers) the large-diameter fibrous material so that the SAP may better adhere. The SAP may also be altered (e.g., SAP slurry which is tacky) to improve adherence. Further, it may be desirable to through-air bond the resulting large-diameter fibrous material so as to set the closed tube formation.

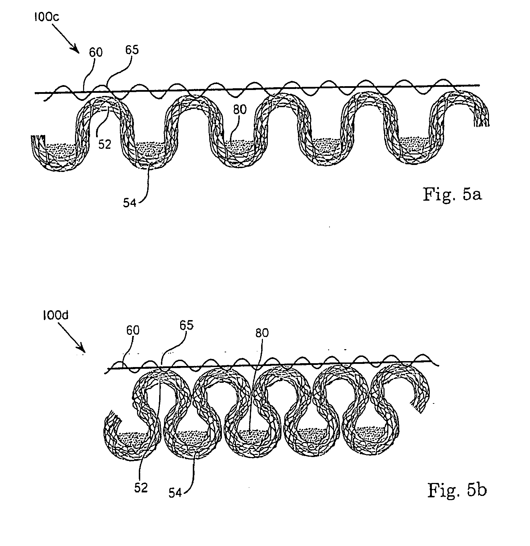

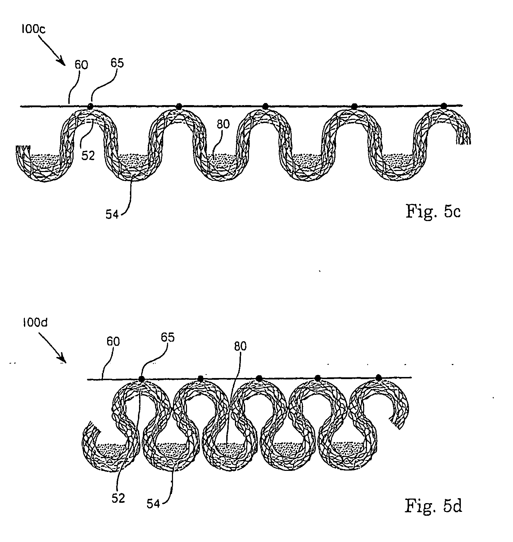

FIG. 5a shows another exemplary resulting product at position 100c within the manufacturing process of FIG. 2a and FIG. 2b, wherein large-diameter fibrous material 10 has peaks 52 and valleys 54 with SAP 80 within the valleys; in addition, an elastic member 60 is affixed to the large-diameter fibrous material. For instance, an elastic member 60 having pre-applied adhesive 65 substantially along its surface may be applied to the tops of peaks 52 in a pre-stretched condition; such that, upon relaxation of the elastic, the peaks will substantially close to form tubes as shown in FIG. 5b. In an alternate embodiment, FIG. 5c shows discrete applications of adhesive 65 being applied to elastic member 60. FIG. 5d shows the resulting, relaxed closed tube product of FIG. 5c. While the disclosed embodiments show the adhesive being pre-applied to the elastic, one skilled in the art would appreciate that the adhesive may also be pre-applied to the target large-diameter fibrous material. Further, it may be desirable for the adhesive to be hydrophilic (e.g., Cycloflex from Natural Starch) so as to allow urine to penetrate and reach the SAP.

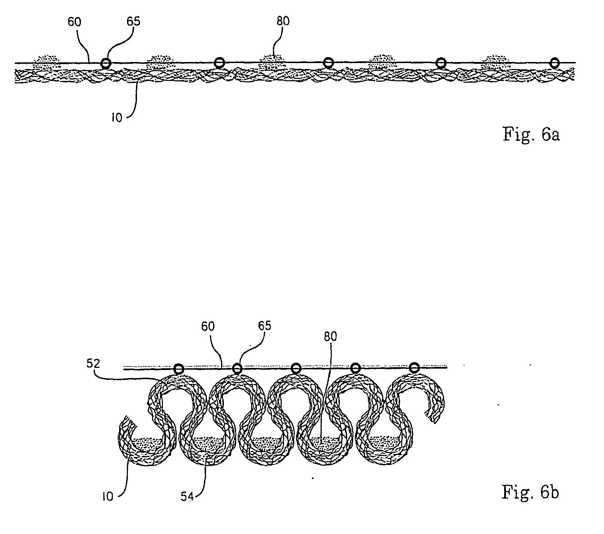

In yet another embodiment, FIG. 6a shows an exemplary large-diameter fibrous material 10 in a substantially planar pre-condition having discrete depositions of SAP 80. Additionally, elastic member 60 having discrete applications of adhesive 65 may be stretched and applied to the large-diameter fibrous material 10. Upon release of the tension, the adhesively-applied elastic member 60 causes the substantially planar large-diameter fibrous material 10 to form peaks 52 and valleys 54 as shown in FIG. 6b.

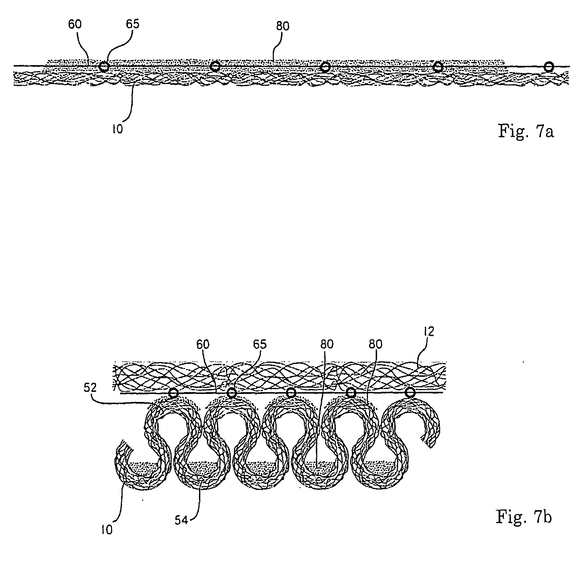

In yet another embodiment, FIG. 7a shows an exemplary large-diameter fibrous material 10 in a substantially planar pre-condition having a substantially continuous deposition of SAP 80. Additionally, elastic member 60 having discrete applications of adhesive 65 may be stretched and applied to the large-diameter fibrous material 10. Upon release of the tension, the adhesively-applied elastic member 60 causes the substantially planar large-diameter fibrous material 10 to form peaks 52 and valleys 54 as shown in FIG. 7b. In this particular example, there are two different resulting locations for the presence of SAP 80, one location being within valleys 54 and another location being on top of peaks 52. Additionally, in this particular example, another layer of large-diameter fibrous material 12 may be subsequently joined to and/or above the peaks so as to protect the deposition of SAP 80 along the peaks.

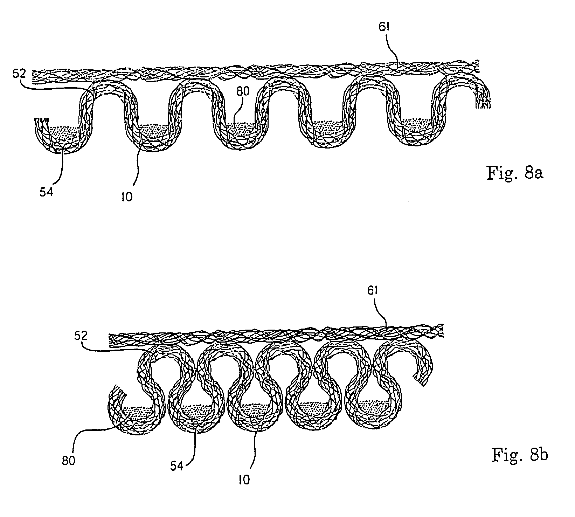

In yet another embodiment, FIG. 8a shows an exemplary large-diameter fibrous material 10 having peaks 52 and valleys 54 with SAP 80 deposited within the valleys. Further, a retractable material 61 (e.g., polyester which retracts upon a stimulus and separately serves as a suitable acquisition layer) may be applied to the peaks by any suitable technique including, but not limited to, adhesive or by its contact in a semi-molten form. Once affixed, and upon the subsequent introduction of some stimulus (e.g., heat), the retractable material 61 retracts such that its overall length is shortened. As a result, the attached peaks are similarly retracted in order to close the valleys 54 to form a tube as shown in FIG. 8b.

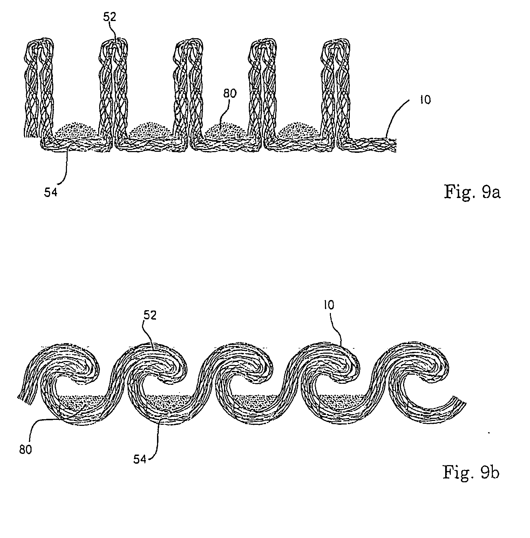

In yet another embodiment, FIG. 9a shows an exemplary large-diameter fibrous material 10 having peaks 52 and valleys 54 with SAP 80 deposited within the valleys. Peaks 52 are pre-formed to have a substantially increased height (such as 30 mm) which lack significant upright integrity. After SAP 80 is deposited in the valleys, and upon some stimulus (e.g., blown air, air from mere movement of large-diameter fibrous material), peaks 52 will begin to fall over in such a manner so as to close the valleys to form a tube as shown in FIG. 9b.

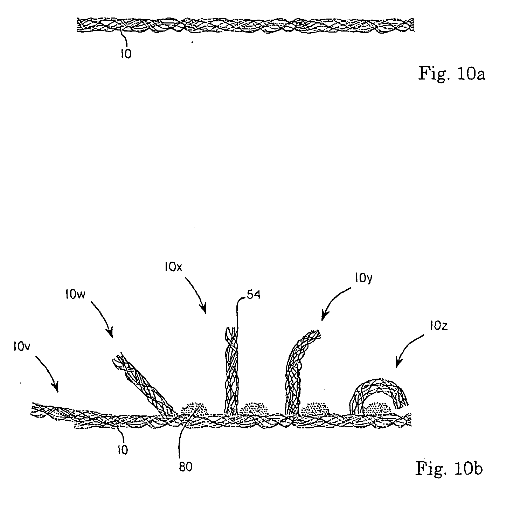

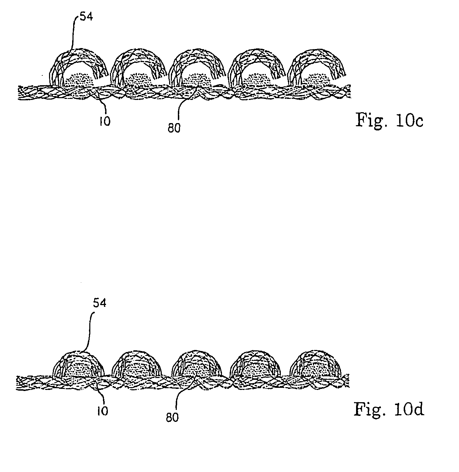

FIG. 10a shows an exemplary large-diameter fibrous material 10 which originally has a substantially planar shape. In response to some stimulus (e.g., blown air, air from mere movement of large-diameter fibrous material), portions of the large-diameter fibrous material will begin to lift upward and back as shown in FIG. 10b. As can be seen in FIG. 10b, a first exemplary position 10v illustrates large-diameter fibrous material being lifted. A second exemplary position 10w illustrates large-diameter fibrous material being lifted at approximately 45°. It is at this point, or at some point nearby, that SAP 80 is deposited into the spacing between the partially vertically lifted large-diameter fibrous material portions. A third exemplary position 10x illustrates large-diameter fibrous material reaching a substantially upright position. A fourth exemplary position 10y illustrates large-diameter fibrous material portion beginning to fold over SAP 80. Lastly, in a fifth exemplary position 10z, large-diameter fibrous material portion has substantially folded over SAP 80 so as to create a substantially closed tube around the SAP 80. Referring now to FIG. 10c, a series of uplifted and fallen back filaments around a series of SAP 80 is shown. FIG. 10d shows the product of FIG. 10c being further consolidated such that the tubes formed from the fallen back filaments are now further closed.

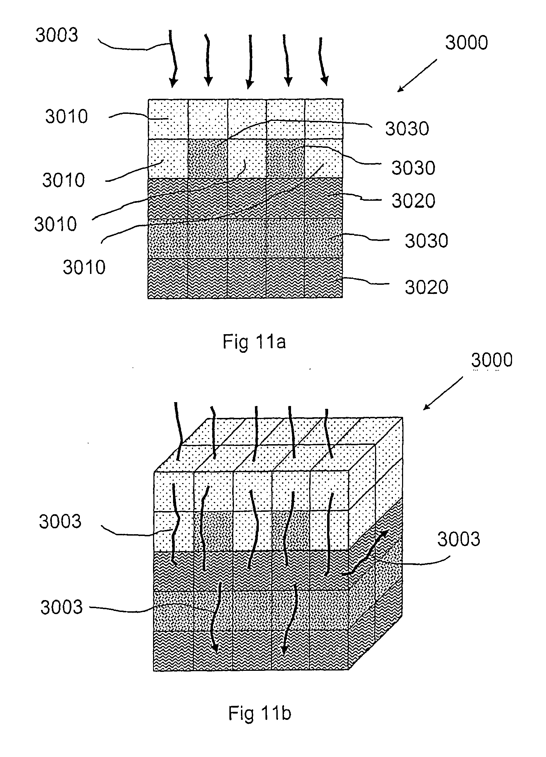

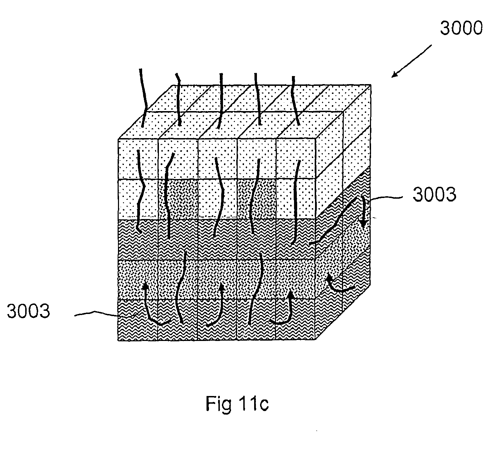

Referring now to FIG. 11a, a two-dimensional schematic is shown to depict one of the benefits of the present invention. More specifically, the novel aspects of the present invention provide for the creation of novel core structure designs. For instance, FIG. 11a shows a two-dimensional schematic view of an absorbent core 3000 having acquisition regions 3010, distribution regions 3020 and storage regions 3030 being selectively placed throughout the core design. Such a designs provides for novel fluid management.

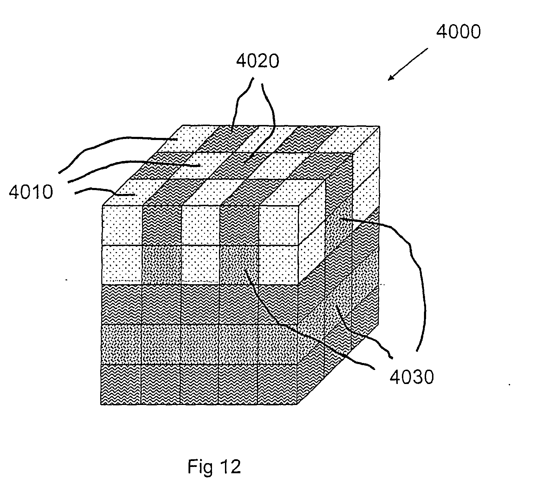

It is well known that conventional absorbent core structures for use in disposable absorbent articles may be made of multiple layers of materials. Further, it is well known that the layers may consist of different types of materials. For example, a conventional absorbent article may be made of: (a) a top layer which serves as an acquisition region for more immediate absorption of exudate from the wearer, (b) an intermediate layer which serves as a storage region for more long-term storage of exudate and (c) a bottom layer which serves as a distribution region for the intended transportation of exudate within the absorbent core structure (e.g., move exudate longitudinally or laterally for greater utilization of diaper). However, such conventional cores often do not permit inter-layer fluid communication. Not only does the present invention provide inter-layer fluid communication, but it provides three-dimensional fluid management as depicted in the series of FIGS. 11a - 11c, wherein the fluid 3003 is moved in accordance with the core design principles disclosed herein. Lastly, the core structure may be designed to have its regions (i.e., acquisition regions 4010, distribution regions 4020 and storage regions 4030) vary in their three-dimensional placement as depicted by absorbent core 4000 in FIG. 12.

While these schematics were shown to illustrate one of the benefits of the present invention, the present invention is not necessarily limited to executions that adhere to the schematics.

All documents cited in the Detailed Description are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

For example, one skilled in the art would appreciate varying degrees of consolidation.

## Claims

1. A method of making an absorbent core structure, comprising:
meltspinning at least a first layer of fibrous material having a plurality of first portions and a plurality of second portions,
depositing a superabsorbent material between the respective first and second portions of the first layer, and
moving the first portions of the first layer with respect to the second portions of the first layer so as to at least substantially encapsulate the deposited superabsorbent material between the respective first and second portions.

2. The method of claim 1, wherein the first and second portions are respectively formed as peaks and valleys and the method further comprises:
depositing the superabsorbent material in at least the valleys.

3. The method of claim 1, wherein the first and second portions are respectively formed as peaks and valleys and the method further comprises:
depositing the superabsorbent material generally uniformly across the peaks and valleys.

4. The method of claim 1, wherein moving the first portions of the first layer with respect to the second portions of the first layer further comprises:
connecting the first layer of fibrous material to a contractable element, and
causing the contractable element to contract.

5. The method of claim 4, wherein the contractable element further comprises a stretched elastic strand.

6. The method of claim 4, wherein the contractable element further comprises a second fibrous layer.

7. The method of claim 1, wherein moving the first portions of the first layer with respect to the second portions of the first layer so as to at least substantially encapsulate the deposited superabsorbent material further comprises:
forming the first and second portions generally into tubular structures with at least a portion of the superabsorbent material received within at least some of the tubular structures.

8. The method of claim 1, further comprising:
depositing the superabsorbent material onto the first layer of fibrous material with the first layer of fibrous material in a generally flat condition.

9. The method of claim 3, further comprising:
depositing the superabsorbent material onto the first layer of fibrous material in discrete, spaced apart amounts.

10. The method of claim 8, further comprising:
depositing the superabsorbent material onto the first layer of fibrous material in a generally continuous layer.

11. The method of claim 1, further comprising:
securing a second layer of fibrous material to the first layer of fibrous material.

12. An apparatus for manufacturing an absorbent core structure, comprising:
a web configuration device operative to receive a layer of fibrous material and form a plurality of peaks and valleys in the layer of fibrous material,
an applicator device positioned downstream of said web configuration device and operative to deposit a superabsorbent material into at least the valleys in the layer of fibrous material, and
an encapsulation device positioned downstream of said applicator device and operative to close the peaks against one another to thereby at least substantially encapsulate the superabsorbent material within the valleys.

13. The apparatus of claim 12, wherein said web configuration device further comprises first and second rotary members engageable with opposite sides of the layer of fibrous material.

14. The apparatus of claim 13, wherein said encapsulation device further comprises third and fourth rotary members engageable with opposite sides of the layer of fibrous material, said third and fourth rotary members being controlled to operate at a lower speed than said first and second rotary members so as to cause the peaks to close against one another.
